(19) Europäisches Patentamt
European Patent Office
Office européen des brevets

(11) Veröffentlichungsnummer: **0 044 002**
**B1**

(12) **EUROPÄISCHE PATENTSCHRIFT**

(45) Veröffentlichungstag der Patentschrift: **20.07.83**

(51) Int. Cl.³: **C 07 C 47/55,** C 07 C 45/44, C 07 C 43/313, C 07 C 41/50, C 07 C 103/22, C 07 C 102/00, C 07 C 121/52, C 07 C 120/10

(21) Anmeldenummer: **81105161.4**

(22) Anmeldetag: **03.07.81**

(54) **Verfahren zur Herstellung von 3-Brom-4-fluor-benzaldehyd-(-acetalen), 3-Brom-4-fluor-benzoesäurenitril und seine Herstellung.**

(30) Priorität: **16.07.80 DE 3026959**

(43) Veröffentlichungstag der Anmeldung: **20.01.82 Patentblatt 82/3**

(45) Bekanntmachung des Hinweises auf die Patenterteilung: **20.07.83 Patentblatt 83/29**

(84) Benannte Vertragsstaaten: **BE CH DE FR GB IT LI NL**

(56) Entgegenhaltungen:
**CH-A-397 635**
**DE-A-2 732 227**
**GB-A-397 124**
**GB-A-1 166 793**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Riebel, Hans-Jochem, Dr., In der Beek 92, D-5600 Wuppertal 1 (DE)**
Erfinder: **Priesnitz, Uwe, Dr., Severinstrasse 58, D-5650 Solingen 1 (DE)**
Erfinder: **Klauke, Erich, Dr., Eichendorffweg 8, D-5068 Odenthal (DE)**

EP 0 044 002 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung von 3-Brom-4-fluor-benzaldehyd(-acetalen), 3-Brom-4-fluor-benzoesäurenitril und seine Herstellung

Die Erfindung betrifft ein neues Verfahren zur Herstellung von 3-Brom-4-fluor-benzaldehyd und dessen Acetalen, sowie 3-Brom-4-fluorbenzoesäurenitril und ein Verfahren zu seiner Herstellung.

Es ist bekannt, dass man 4-Fluor-3-phenoxy-benzaldehyd, ein Zwischenprodukt für pestizid wirksame Pyrethroide, erhält, wenn man 4-Fluor-3-phenoxy-benzylbromid mit Hexamethylentetramin umsetzt und das Produkt dieser Umsetzung mit Säuren erhitzt (vergleiche DE-OS 2709264). Die Ausbeute ist jedoch bei dieser Synthesemethode, wie auch bei der Herstellung der Ausgangsverbindung aus 4-Fluor-3-phenoxy-toluol und N-Brom-succinimid, nicht voll befriedigend.

Weiter sind 3-Brom-4-fluor-benzaldehyd und Acetale hiervon sowie 4-Fluor-3-phenoxy-benzaldehyd-acetale als neue Zwischenprodukte zur Herstellung von 4-Fluor-3-phenoxy-benzaldehyd Gegenstand einer älteren, nicht vorveröffentlichten Patentanmeldung (vgl. DE-OS 2933979).

Ferner ist bekannt geworden, dass man bestimmte aromatische Aldehyde erhält, wenn man die entsprechenden Nitrile mit Ameisensäure in Gegenwart von feuchtem Raney-Nickel umsetzt (vgl. J. Chem. Soc. (London) 1964, 5880–1).

Gegenstand der vorliegenden Patentanmeldung sind

(1) ein Verfahren zur Herstellung von 3-Brom-4-fluor-benzaldehyd(-acetalen) der Formel I

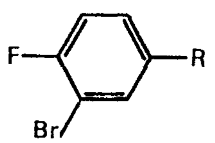

(I)

in welcher
R für $-CHO$ oder $-CH(OR^1)_2$ steht,
wobei $R^1$ für Methyl oder Ethyl steht,
dadurch gekennzeichnet, dass man 3-Brom-4-fluor-benzoesäurenitril der Formel II

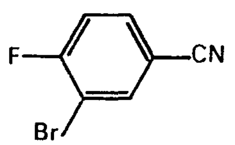

(II)

mit Ameisensäure in Gegenwart eines Metallkatalysators bei Temperaturen zwischen 0 und 150°C umsetzt und gegebenenfalls den hierbei gebildeten Aldehyd der Formel I a

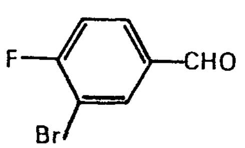

(Ia)

nach üblichen Methoden acetalisiert;

(2) das neue 3-Brom-4-fluor-benzoesäurenitril der Formel II

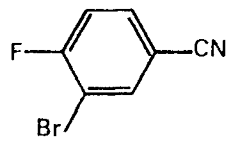

(II)

(3) ein Verfahren zur Herstellung von 3-Brom-4-fluor-benzoesäurenitril der Formel II (oben), dadurch gekennzeichnet, dass man 3-Brom-4-fluor-benzoesäureamid der Formel III

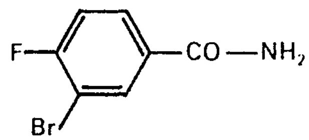

(III)

nach üblichen Methoden dehydratisiert.

Das neue 3-Brom-4-fluor-benzoesäureamid der Formel III

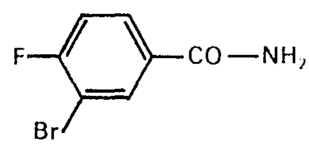

(III)

erhält man, indem man 3-Brom-4-fluor-benzoesäurehalogenide der Formel IV

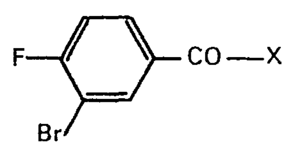

(IV)

in welcher
X für Fluor, Chlor oder Brom steht,
mit Ammoniak, gegebenenfalls in Gegenwart eines Verdünnungsmittels umsetzt.

Überraschenderweise kann 4-Fluor-3-phenoxy-benzaldehyd über die vorstehend aufgeführten neuen Zwischenprodukte auf einfachere Weise und in besserer Ausbeute als nach dem oben erwähnten bekannten Verfahren hergestellt werden.

Das unter (1) dargelegte neue Verfahren («Verfahren (1)») kann bei Verwendung von Raney-Nickel als Katalysator und von Orthoameisensäuretriethylester in Ethanol als Acetalisierungsmittel durch folgendes Reaktionsschema skizziert werden:

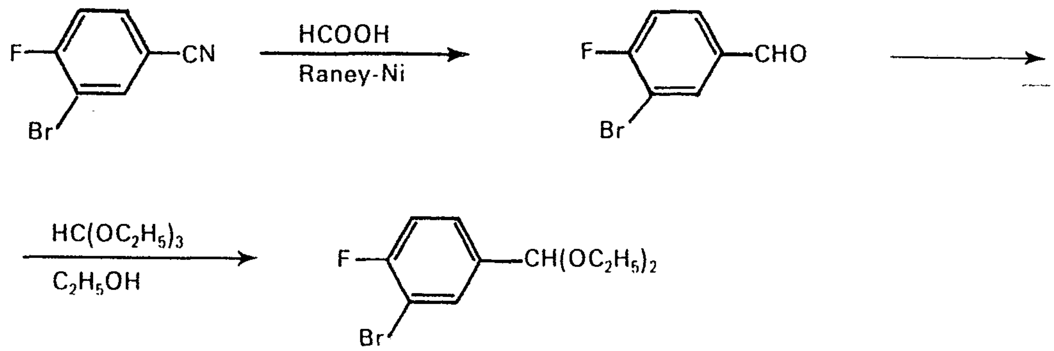


Verfahren (1) wird in Gegenwart eines Katalysators durchgeführt. Es können die üblicherweise bei katalytischen Hydrierungen eingesetzten Metallkatalysatoren verwendet werden. Als bevorzugter Katalysator sei Raney-Nickel genannt. Die Katalysatoren können in wasserfeuchtem Zustand eingesetzt werden.

Die Temperatur wird bei Verfahren (1) zwischen 0 und 150°C, vorzugsweise zwischen 50 und 120°C gehalten. Das Verfahren wird bei Normaldruck oder geringfügig erhöhtem oder vermindertem Druck durchgeführt.

Ameisensäure wird bei Verfahren (1) als Reaktand und Verdünnungsmittel verwendet und dementsprechend in hohem Überschuss, vorzugsweise in einer Menge zwischen 10 und 50 Mol je Mol Ausgangsverbindung der Formel (II) eingesetzt.

Zur Durchführung des erfindungsgemässen Verfahrens (1) wird in einer bevorzugten Ausführungsform der Katalysator in der Ameisensäure suspendiert, zu dieser Suspension wird das 3-Brom-4-fluor-benzoesäurenitril (II) gegeben und die Reaktionsmischung wird mehrere Stunden erhitzt. Die Aufarbeitung kann nach üblichen Methoden erfolgen, beispielsweise durch Verdünnen mit Wasser, Filtrieren, Extrahieren des Filters mit einem mit Wasser praktisch nicht mischbaren organischen Lösungsmittel, wie z.B. Toluol, Trocknen der Extrakte, Filtrieren und Destillieren des Filtrats unter vermindertem Druck, wobei man 3-Brom-4-fluor-benzaldehyd als farblose Flüssigkeit erhält.

Die 3-Brom-4-fluor-benzaldehyd-dimethyl- bzw. diethylacetale sind bisher noch nicht beschrieben. Sie können ausser nach dem oben angegebenen Verfahren auch nach an sich bekannten Verfahren hergestellt werden. Man erhält sie beispielsweise, wenn man 3-Brom-4-fluor-benzaldehyd mit Orthoameisensäure-trimethylester bzw. -triethylester in Gegenwart von Verdünnungsmitteln, wie z.B. Toluol und Methanol bzw. Ethanol und gegebenenfalls in Gegenwart eines Ionenaustauschers als Katalysator bei Temperaturen zwischen 0 und 100°C, vorzugsweise zwischen 10 und 80°C umsetzt. Die Aufarbeitung kann hiernach ebenfalls nach üblichen Methoden durchgeführt werden, beispielsweise durch Filtrieren, Waschen des Filtrats mit verdünnter Kaliumcarbonatlösung und Destillieren unter vermindertem Druck.

Das bei Verfahren (1) als Ausgangsverbindung zu verwendende neue 3-Brom-4-fluor-benzoesäurenitril (II) kann aus 3-Brom-4-fluor-benzoesäureamid (III) nach an sich bekannten Dehydratisierungsverfahren hergestellt werden (vgl. Methodicum Chimicum, Band 6 (1974), S. 654–656).

Man erhält das Nitril (II) beispielsweise, wenn man 3-Brom-4-fluor-benzoesäureamid bei ca. 25°C zu überschüssigem Thionylchlorid gibt, die Mischung bis zum Ende der Gasentwicklung unter Rückfluss zum Sieden erhitzt und destilliert.

Das neue 3-Brom-4-fluor-benzoesäureamid (III) kann durch Umsetzung von 3-Brom-4-fluorbenzoesäurehalogeniden der Formel IV (oben) mit Ammoniak nach an sich bekannten Methoden hergestellt werden (vgl. Methodicum Chimicum, Band 6 (1974), S. 686).

Man erhält das Amid (III) beispielsweise, wenn man 3-Brom-4-fluor-benzoesäurefluorid (und/oder das Säurechlorid und/oder das Säurebromid der Formel IV) langsam zu einer auf 30 bis 60°C erwärmten wässrigen Ammoniaklösung gibt und die Mischung einige Stunden rührt. Das Produkt der Formel (III) kristallisiert aus und kann durch Filtration isoliert werden.

3-Brom-4-fluor-benzoesäurehalogenide der Formel IV (oben), welche als Ausgangsstoffe zu verwenden sind, sind Gegenstand einer nicht zum vorveröffentlichten Stand der Technik gehörenden Patentanmeldung (vgl. P 2915738/Le A 19590).

Man erhält beispielsweise ein Gemisch aus 3-Brom-4-fluor-benzoesäurefluorid und -bromid, wenn man 4-Chlor-benzoylchlorid durch Umsetzung mit Kaliumfluorid in 4-Fluorbenzoylfluorid umwandelt und letzteres anschliessend bromiert.

4-Chlor-benzoylchlorid wird mit Kaliumfluorid beispielsweise in Tetramethylensulfon bei Temperaturen zwischen 200 und 220°C umgesetzt und das Reaktionsgemisch destillativ aufgearbeitet. Man erhält 4-Fluor-benzoylfluorid vom Siedepunkt 53°C/20 mBar (Brechungsindex: $n_D^{20}$ = 1,4792).

4-Fluor-benzoylfluorid wird mit elementarem Brom in Gegenwart von 1% Eisen(III)chlorid bei 70 bis 75°C umgesetzt. Bei einem Ansatz von 1 Mol erhält man nach Destillation 40 g unverändertes Ausgangsmaterial zurück und 182 g eines Gemisches aus 3-Brom-4-fluor-benzoylfluorid (Siedepunkt: 82–83°C/15 mBar; Brechungsindex: $n_D^{20}$ = 1,5315; Schmelzpunkt: 32–34°C) und 3-Brom-4-fluor-benzoylbromid (Siedepunkt: 123°C/15 mBar; Schmelzpunkt: 35–37°C).

3-Brom-4-fluor-benzaldehyde bzw. deren Acetale der Formel I (oben) können zur Herstellung von 3-Phenoxy-4-fluor-benzaldehyd, welcher als Zwischenprodukt für Insektizide bekannt ist (vgl. DE-OS 2 709 264), verwendet werden.

Die Herstellung von 3-Phenoxy-4-fluor-benzaldehyd kann beispielsweise bei Verwendung von 3-Brom-4-fluor-benzaldehyd-diethylacetal und Kaliumphenolat als Ausgangsstoffen in einer ersten Stufe und bei einer als zweite Stufe durchzuführenden Acetalspaltung mit einer Säure, wie z.B. Salzsäure, durch folgendes Formelschema skizziert werden:

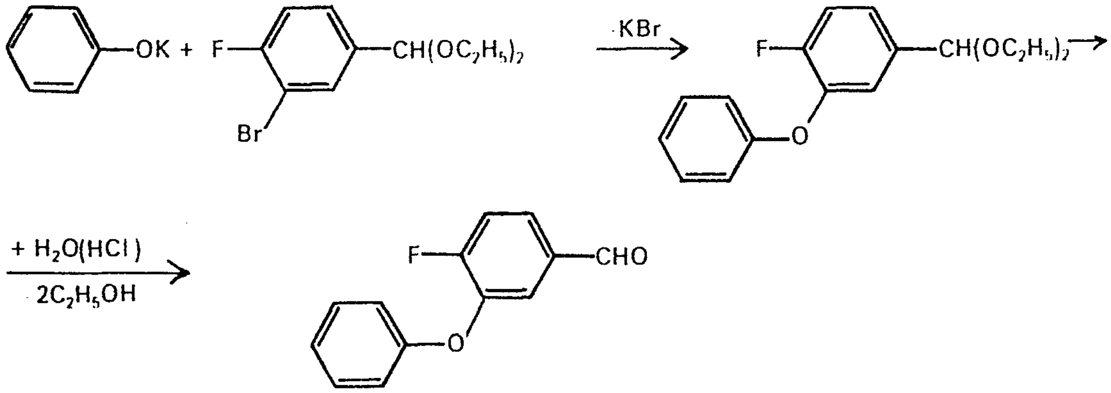


Alkali- bzw. Erdalkaliverbindungen von Phenolen, welche hierbei als Ausgangsstoffe verwendet werden können, sind z.B. Natrium-, Kalium- und Magnesium-phenolat.

Als Katalysatoren werden Kupfer oder Kupferverbindungen verwendet. Als Beispiele hierfür seien Kupfer, Kupfer-(I)-oxid, Kupfer-(II)-oxid, Kupfer-(I)-chlorid und Kupfer-(I)-bromid genannt.

Als Verdünnungsmittel werden vorzugsweise aprotisch polare Solventien verwendet. Beispiele hierfür sind Dimethylformamid, Dimethylacetamid, N-Methylpyrrolidon, Dimethylsulfoxid, Tetramethylensulfon, Hexamethylphosphorsäuretriamid und Bis-(2-methoxyethyl)-ether (Diglyme). Letzteres wird besonders bevorzugt.

Die Reaktionstemperatur wird zwischen 100 und 200°C, vorzugsweise zwischen 130 und 170°C gehalten. Das Verfahren wird gewöhnlich unter Normaldruck durchgeführt.

Auf 1 Mol 3-Brom-4-fluor-benzaldehyd-acetal der Formel (Ib) setzt man 1 bis 2 Mol, vorzugsweise 1,2 bis 1,8 Mol Phenolat, 0,01 bis 0,5 Mol, vorzugsweise 0,1 bis 0,5 Mol Kupferkatalysator und 100 bis 500 ml Verdünnungsmittel ein.

In einer bevorzugten Ausführungsform der oben skizzierten Umsetzung zur Herstellung von 4-Fluor-3-phenoxy-benzaldehyd-acetalen wird das Phenolat in einem der oben angegebenen Verdünnungsmittel zusammen mit dem Kupferkatalysator vorgelegt und das Gemisch wird auf die Reaktionstemperatur aufgeheizt. Dann wird das 3-Brom-4-fluorbenzaldehyd-acetal zugetropft und das Reaktionsgemisch einige Stunden nachgerührt. Die Aufarbeitung kann nach üblichen Methoden erfolgen, beispielsweise durch Abdestillieren des Verdünnungsmittels unter vermindertem Druck, Lösen des Rückstandes in Toluol, Filtrieren, Waschen des Filtrats mit verdünnter Natronlauge und Destillieren unter vermindertem Druck. Man erhält so die 3-Phenoxy-4-fluor-benzaldehyd-acetale als farblose Flüssigkeiten.

Die Verseifung der Acetale zu 3-Phenoxy-4-fluor-benzaldehyd kann nach üblichen Methoden durchgeführt werden. In einer bevorzugten Verfahrensweise werden die Acetale mit einer verdünnten Mineralsäure, wie z.B. Salzsäure oder Schwefelsäure, vermischt und mehrere Stunden bei Temperaturen zwischen 20 und 60°C gerührt. Zur Aufarbeitung wird mit einem mit Wasser praktisch nicht mischbaren organischen Lösungsmittel, wie z.B. Toluol, extrahiert, die Extrakte werden mit Natriumhydrogencarbonatlösung und Wasser gewaschen und durch Destillation unter vermindertem Druck vom Lösungsmittel befreit. 3-Phenoxy-4-fluor-benzaldehyd verbleibt hierbei als öliger Rückstand.

## Herstellungsbeispiele

### Beispiel 1:

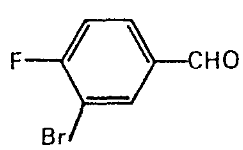

17,0 g einer 50%igen alkalischen Raney-Nickel-Suspension werden in einer Stickstoffatomosphäre abgesaugt und neutral gewaschen. Das nach dieser Methode erhaltene Raney-Nickel wird in 150 ml Ameisensäure suspendiert. Zu dieser Suspension werden 20,0 g (0,1 Mol) 3-Brom-4-fluor-benzonitril gegeben. Man erwärmt 3-5 Stunden auf 80-90°C, kühlt dann ab und giesst das Reaktionsgemisch in 300 ml Wasser. Nach einer Filtration erfolgt zweimalige Extraktion mit je 200 ml Toluol. Die Toluolphase wird über Natriumsulfat getrocknet und dann eingeengt. Man erhält 17,2 g (85% der Theorie) 3-Brom-4-fluorbenzaldehyd mit dem Siedepunkt 63-65°C/0,3 mmHg.

Beispiel 2:

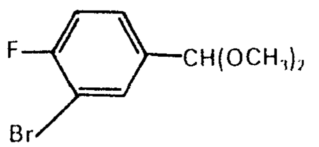

Eine Lösung von 101,5 g (0,5 Mol) 3-Brom-4-fluorbenzaldehyd in 200 ml Toluol versetzt man mit 1,5 g an der Luft getrocknetem Ionenaustauscher SPC 118 (H-Form eines Kationenaustauschers, stark sauer, makroporös), 16 g (0,5 Mol) Methanol und 58,5 g (0,55 Mol) Orthoameisensäure-trimethylester, rührt 1 Stunde ohne Bad nach und erwärmt dann 2 Stunden auf 50°C. Das Reaktionsgemisch wird abgekühlt, vom Ionenaustauscher filtriert und mit 200 ml 5%iger Kaliumcarbonatlösung gewaschen. Dann zieht man das Lösungsmittel ab und destilliert den Rückstand im Vakuum. Man erhält auf diese Weise 118 g (95% der Theorie) 3-Brom-4-fluorbenzaldehyddimethylacetat als farblose Flüssigkeit mit dem Siedepunkt 68°C/0,1 mmHg.

Beispiel 3:

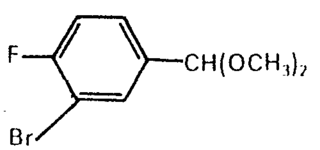

Eine Mischung aus 85 g mit Wasser ausgewaschenem Raney-Nickel, 500 ml Ameisensäure und 100 g (0,5 Mol) 3-Brom-4-fluor-benzonitril wird 24 Stunden bei 90–100°C unter einer Schutzgasatmosphäre (Stickstoff oder Argon) gerührt. Nach Abkühlen auf Raumtemperatur wird mit 1 l Wasser versetzt und zweimal mit je 300 ml Toluol extrahiert. Die vereinigten Toluollösungen werden mit 200 ml Wasser, 200 ml gesättigter Natriumhydrogencarbonatlösung und wieder mit 200 ml Wasser gewaschen und durch Andestillieren getrocknet. Danach versetzt man die Lösung mit 1,5 g an der Luft getrocknetem Ionenaustauscher SPC 118, 16 g (0,5 Mol) Methanol und 58,5 g (0,55 Mol) Orthoameisensäuretrimethylester, rührt 1 Stunde ohne Bad nach und erwärmt dann 2 Stunden auf 50°C. Das Reaktionsgemisch wird abgekühlt, vom Ionenaustauscher filtriert und mit 200 ml 5%iger Kaliumcarbonatlösung gewaschen. Dann zieht man das Lösungsmittel ab und destilliert den Rückstand im Vakuum. Man erhält auf diese Weise 93 g (75% der Theorie) 3-Brom-4-fluor-benzaldehyddimethylacetal in Form einer farblosen Flüssigkeit mit dem Siedepunkt 67–68°C/0,1 mmHg.

Beispiel 4:

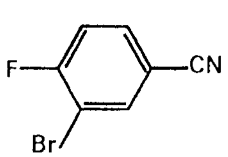

765 g (3,5 Mol) 3-Brom-4-fluor-benzoesäureamid werden bei 25°C zu 1150 g (9,7 Mol) Thionylchlorid gegeben und das Gemisch wird unter Rühren auf 85 bis 90°C unter Rückfluss erhitzt, bis praktisch keine Gasentwicklung mehr erfolgt. Dann wird das überschüssige Thionylchlorid abdestilliert und der Rückstand an einer kurzen Kolonne fraktioniert. Man erhält 639 g (91% der Theorie) 3-Brom-4-fluor-benzonitril vom Siedepunkt 115–116°C/20 Torr.

Beispiel 5:

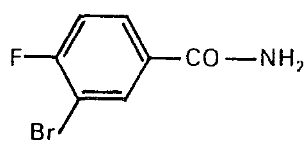

800 g (3,6 Mol) 3-Brom-4-fluor-benzoylfluorid werden zu 760 g 25%iger wässriger Ammoniaklösung (11 Mol $NH_3$), welche man mit weiteren 760 ml Wasser verdünnt hat so eindosiert, dass durch die schwach exotherme Reaktion eine Innentemperatur von 40 bis 50°C aufrechterhalten wird. Nach 30 Minuten Nachrühren wird das kristalline Produkt durch Filtration isoliert, mit Wasser gewaschen und getrocknet. Man erhält 765 g (97% der Theorie) 3-Brom-4-fluor-benzoesäureamid vom Schmelzpunkt 131–133°C.

Beispiel 6:

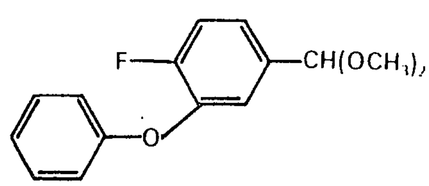

95 ml Diethylenglykoldimethylether (Diglyme) werden mit 0,4 g Kupfer-I-bromid und 43,5 g (0,375 Mol) Natriumphenolat versetzt. Die Temperatur des Gemisches steigt bis ca. 80°C an. Zur Entwässerung werden bei Normaldruck 15 ml Diglyme (Siedepunkt 160°C) abdestilliert. Dann tropft man bei 150–155°C innerhalb von 10 Min. 57,3 g (0,23 Mol) 3-Brom-4-fluor-benzaldehyddimethylacetal zu und rührt das Reaktionsgemisch 7 Stunden bei 155°C nach.

Nach GC ist dann alles Vorprodukt umgesetzt. Anschliessend destilliert man bei 40°C/10 Torr das Lösungsmittel ab, löst den Rückstand in 300 ml Toluol und saugt nach Zugabe von 10 g Filtrierhilfe Celite 545 vom anorganischen Material ab. Das Filtrat wird 2 mal mit je 10 ml 5%iger Natronlauge gewaschen, dann zieht man das Lösungsmittel im Vakuum ab und destilliert den Rückstand im Vakuum. Man erhält so 45,5 g (75% der Theorie) 3-Phenoxy-4-fluorbenzaldehyddimethylacetal in Form eines farblosen Öles mit dem Siedepunkt 128–130°C/0,1 mmHg.

Beispiel 7:

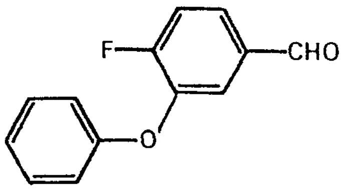

Eine Mischung aus 100 ml 20%iger Schwefelsäure und 52,4 g (0,2 Mol) 3-Phenoxy-4-fluorbenzaldehyddimethylacetal wird 4 Stunden bei 45–50°C gerührt und dann auf Raumtemperatur abgekühlt. Der Aldehyd wird in 100 ml Toluol aufgenommen und mit 100 ml gesättigter Natriumhydrogencarbonatlösung und mit 100 ml Wasser gewaschen. Dann destilliert man das Lösungsmittel im Vakuum vollständig ab. Zurück bleiben 42 g (97% der Theorie) 3-Phenoxy-4-fluorbenzaldehyd, der nach GC-Analyse einen Gehalt von 96,6% besitzt.

**Patentansprüche**

1) Verfahren zur Herstellung von 3-Brom-4-fluorbenzaldehyd(-acetalen) der Formel I

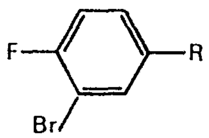
(I)

in welcher
R für -CHO oder $-CH(OR^1)_2$ steht,
wobei $R^1$ für Methyl oder Ethyl steht,
dadurch gekennzeichnet, dass man 3-Brom-4-fluor-benzoesäurenitril der Formel II

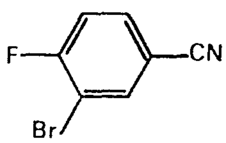
(II)

mit Ameisensäure in Gegenwart eines Metallkatalysators bei Temperaturen zwischen 0 und 150°C umsetzt und gegebenenfalls den hierbei gebildeten Aldehyd der Formel Ia

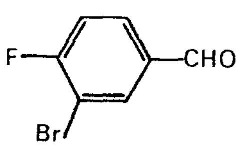
(Ia)

nach üblichen Methoden acetalisiert.

2) 3-Brom-4-fluor-benzoesäurenitril der Formel II

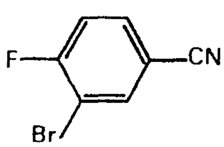
(II)

3) Verfahren zur Herstellung von 3-Brom-4-fluorbenzoesäurenitril der Formel II

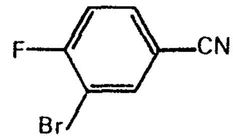
(II)

dadurch gekennzeichnet, dass man 3-Brom-4-fluor-benzoesäureamid der Formel III

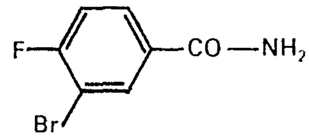
(III)

nach üblichen Methoden dehydratisiert.

**Revendications**

1. Procédé de préparation de 3-bromo-4-fluoro-benzaldéhyde (et de ses acétals) de formule I:

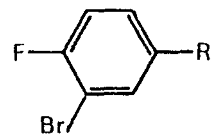
(I)

dans laquelle
R représente -CHO ou $-CH(OR^1)_2$, $R^1$ représentant un groupe méthyle ou éthyle,
caractérisé en ce qu'on fait réagir le nitrile d'acide 3-bromo-4-fluoro-benzoïque de formule II:

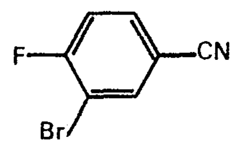
(II)

avec l'acide formique en présence d'un catalyseur métallique à des températures comprises entre 0 et 150°C et on soumet éventuellement l'aldéhyde ainsi formé de formule Ia:

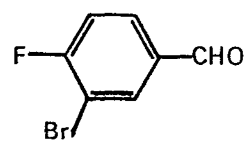
(Ia)

à une acétalisation selon les méthodes habituelles.

2. Le nitrile d'acide 3-bromo-4-fluoro-benzoïque de formule II:

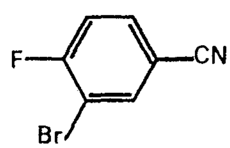
(II)

3. Procédé de préparation du nitrile d'acide 3-bromo-4-fluoro-benzoïque de formule II:

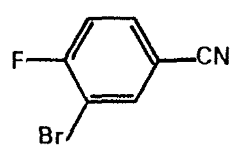
(II)

caractérisé en ce qu'on soumet l'amide d'acide 3-bromo-4-fluoro-benzoïque de formule III:

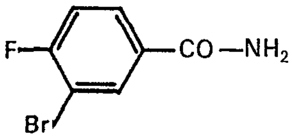

(III)

à une déshydratation selon les méthodes habituelles.

**Claims**

1. Process for the preparation of 3-bromo-4-fluoro-benzaldehyde and 3-bromo-4-fluoro-benzaldehyde acetals of the formula I

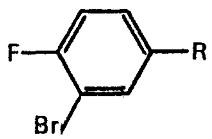

(I)

in which
R represents -CHO or $-CH(OR^1)_2$,
in which $R^1$ represents methyl or ethyl,
characterised in that 3-bromo-4-fluoro-benzonitrile of the formula II

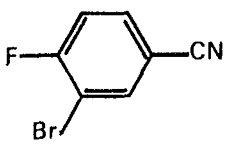

(II)

is reacted with formic acid in the presence of a metal catalyst at temperatures between 0 and 150°C and, if appropriate, the aldehyde of the formula Ia

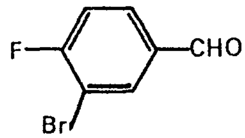

(Ia)

which is thus formed is acetalised by conventional methods.

2. 3-Bromo-4-fluoro-benzonitrile of the formula II

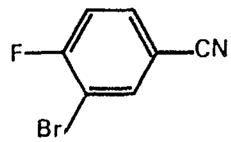

(II)

3. Process for the preparation of 3-bromo-4-fluorobenzonitrile of the formula II

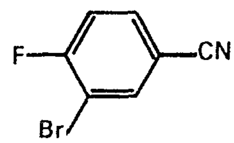

(II)

characterised in that 3-bromo-4-fluoro-benzoic acid amide of the formula III

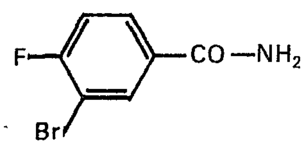

(III)

is dehydrated by conventional methods.